# EUROPEAN PATENT SPECIFICATION

(11) **EP 4 422 642 B1**
(45) Date of publication and mention of the grant of the patent: **04.02.2026**
(21) Application number: 22801892.5
(22) Date of filing: 24.10.2022
(51) Int. Cl.: A61K 31/7048, A61P 35/00

(54) **RUTINOSYL-4-METHYLUMBELLIFERONE FOR THE TREATMENT OF HEPATOCELLULAR CARCINOMA**
RUTINOSYL-4-METHYLUMBELLIFERON ZUR BEHANDLUNG VON HEPATOZELLULÄREM KARZINOM
RUTINOSYL-4-METHYLUMBELLIFÉRONE POUR LE TRAITEMENT DU CARCINOME HÉPATOCELLULAIRE

(30) Priority: 26.10.2021 US 202163271808 P
(43) Date of publication of application: 04.09.2024
(73) Proprietor: Consejo Nacional de Investigaciones Científicas y Técnicas (CONICET), Buenos Aires C1425FQB (AR); Asociación Civil de Estudios Superiores - Universidad Austral, Pilar, B1630FHB (AR); Universidad Nacional de La Pampa, Santa Rosa, L6300 (AR)
(72) Inventor: BRECCIA, Javier Darío, Santa Rosa, 6300 (AR); WEIZ, Gisela, Santa Rosa, 6300 (AR); MOLEJÓN, María Inés, Santa Rosa, 6300 (AR); MAZZAFERRO, Laura Soledad, Santa Rosa, 6300 (AR); MALVICINI, Mariana, Rodríguez, 1748 (AR); MAZZOLINI RIZZO, Guillermo Daniel, Escobar, 1609 (AR)
(74) Representative: Icosa
(86) International application number: PCT/IB2022/060197
(87) International publication number: WO 2023/073531

(56) References cited:
- LAURA S MAZZAFERRO ET AL: "Transglycosylation specificity ofsp. -rhamnosyl--glucosidase and its application to the synthesis of the new fluorogenic substrate 4-methylumbelliferyl-rutinoside", CARBOHYDRATE RESEARCH, PERGAMON, GB, vol. 347, no. 1, 11 November 2011 (2011-11-11), pages 69 - 75, XP028435025, ISSN: 0008-6215, [retrieved on 20111119], DOI: 10.1016/J.CARRES.2011.11.008
- SUKOWATI CAECILIA H. C. ET AL: "Hyaluronic acid inhibition by 4-methylumbelliferone reduces the expression of cancer stem cells markers during hepatocarcinogenesis", SCIENTIFIC REPORTS, vol. 9, no. 1, 11 March 2019 (2019-03-11), XP055965279, Retrieved from the Internet <URL:http://www.nature.com/articles/s41598-019-40436-6> DOI: 10.1038/s41598-019-40436-6
- PICCIONI F. ET AL: "Antitumor effects of hyaluronic acid inhibitor 4-methylumbelliferone in an orthotopic hepatocellular carcinoma model in mice", GLYCOBIOLOGY, vol. 22, no. 3, 28 October 2011 (2011-10-28), US, pages 400 - 410, XP093014685, ISSN: 0959-6658, DOI: 10.1093/glycob/cwr158
- THUAN NGUYEN HUY ET AL: "Recent biotechnological progress in enzymatic synthesis of glycosides", JOURNAL OF INDUSTRIAL MICROBIOLOGY & BIOTECHNOLOGY, BASINGSTOKE, GB, vol. 40, no. 12, 5 September 2013 (2013-09-05), pages 1329 - 1356, XP035330765, ISSN: 1367-5435, [retrieved on 20130905], DOI: 10.1007/S10295-013-1332-0
- FAN N ET AL: "Glycosylation and sulfation of 4-methylumbelliferone byGliocladium deliquescensNRRL 1086", APPLIED BIOCHEMISTRY AND MICROBIOLOGY, NEW YORK, NY, US, vol. 53, no. 1, 21 February 2017 (2017-02-21), pages 85 - 93, XP036163034, ISSN: 0003-6838, [retrieved on 20170221], DOI: 10.1134/S0003683817010033
- WEIZ GISELA ET AL: "Glycosylated 4-methylumbelliferone as a targeted therapy for hepatocellular carcinoma", LIVER INTERNATIONAL, vol. 42, no. 2, 10 December 2021 (2021-12-10), GB, pages 444 - 457, XP055965258, ISSN: 1478-3223, Retrieved from the Internet <URL:https://onlinelibrary.wiley.com/doi/full-xml/10.1111/liv.15084> DOI: 10.1111/liv.15084
- WEIZ GISELA ET AL: "Abstract 5447: Enhanced antitumoral effect of the glycosylated 4-methylumbelliferone in hepatocellular carcinoma", CANCER RESEARCH, vol. 82, no. 12_Supplement, 15 June 2022 (2022-06-15), pages 5447, XP093014739, Retrieved from the Internet <URL:https://aacrjournals.org/cancerres/article/82/12_Supplement/5447/702782/Abstract-5447-Enhanced-antitumoral-effect-of-the> DOI: https://doi.org/10.1158/1538-7445.AM2022-5447

## Description

### FIELD OF THE INVENTION

The present invention generally relates to compositions and treatment of cancer. More particularly, the present invention refers to compositions and treatment of hepatocellular carcinoma. Specifically, the present invention refers to a glycoconjugate useful for the treatment of hepatocellular carcinoma. Yet more specifically, the glycoconjugate is a rutinosylated coumarin derivative. This compound has proven herein to be efficient in experimental models of hepatocellular carcinoma both *in vitro* and *in vivo.*

### BACKGROUND OF THE INVENTION

Cancer is a major public health problem worldwide. Gastrointestinal cancers (GC) account for approximately one-third of the total global cancer incidence and mortality. GCs encompass a variety of diseases including esophageal cancer, colorectal (CRC), pancreatic (PC), hepatic carcinoma (HCC). Only CRC is listed in the top 10 for incidence rate of tumor; however, four gastrointestinal cancers, including colorectal, pancreatic, hepatic, and biliary tract, and esophageal cancers, are in the top 10 for death rates from tumors in the USA.

Additionally, there are four gastrointestinal carcinomas - colorectal, gastric, pancreatic, and hepatic carcinomas - in the top five for death rates in Japan. Specifically, HCC constitutes the third leading cause of cancer-related death worldwide. Incidence and mortality of hepatocarcinoma are constantly increasing (Bray et al. Global Cancer Statistics 2018: GLOBOCAN Estimates of Incidence and Mortality Worldwide for 36 Cancers in 185 Countries. CA: Cancer J Clin. 2018; 68:394-424).

Unfortunately, curative treatments such as surgery, liver transplantation, or ablation can only be applied in less than 30% of the patients with HCC. The current therapeutic options for advanced HCC are tyrosine kinase inhibitors (TKI) such as first-line sorafenib and lenvatinib or regorafenib, cabozantinib, or second-line ramucirumab (Bruix J, Reig M, Sherman M. (2016) Evidence-Based Diagnosis, Staging, and Treatment of Patients with Hepatocellular Carcinoma. Gastroenterology 150:835-53.; Llovet JM, Ricci S, Mazzaferro V, et al. (2008) Sorafenib in advanced hepatocellular carcinoma. N Engl J Med 359:378-90). However, many patients are resistant to these therapies. Hence, new, more powerful, and more specific therapeutic approaches are required.

During hepatocarcinogenesis, the accumulation of extracellular matrix (ECM) occurs in the liver parenchyma. This excessive deposition of ECM culminates in major changes in liver architecture acting as a reservoir for pro-inflammatory and pro-fibrogenic mediators leading to liver fibrosis. Hepatic stellate cells (HSCs) are the key fibrogenic effector cell type in liver and the main ECM-producing cells. When hepatic injury persists, HSCs become activated and are transformed into myofibroblast-like cells (Wynn TA. (2007) Common and unique mechanisms regulate fibrosis in various fibroproliferative diseases. Journal of Clinical Investigation. 117(3):524-9; Friedman SL. Hepatic stellate cells: protean, multifunctional, and enigmatic cells of the liver. Physiol Rev. 2008;88(1):125-172).

Hyaluronic acid (hyaluronan, HA) is one of the markers of fibrosis (Toole BP. (2004) Hyaluronan : from extracellular glue to pericellular cue. Nature Reviews Cancer 4(July):528-39). It is a linear, large and ubiquitous non-sulfated glycosaminoglycan of the ECM produced by three hyaluronan synthases (HAS1, 2, and 3) (Pares et al. (1999) Serum hyaluronate reflects hepatic fibrogenesis in alcoholic liver disease and is useful as a marker of fibrosis; Hepatology, 24(6):1399-403). The coumarin 4-Methylumbelliferone (4MU) has been described as an inhibitor of HA synthesis in many studies (Mima K, et al. (2014) Preoperative serum hyaluronic acid level as a prognostic factor in patients undergoing hepatic resection for hepatocellular carcinoma. British Journal of Surgery 101(3):269-76; Piccioni F, et al. (2012) Antitumor effects of hyaluronic acid inhibitor 4-methylumbelliferone in an orthotopic hepatocellular carcinoma model in mice. Glycobiology 22(3):400-10; Kakizaki I, et al. (2004) A novel mechanism for the inhibition of hyaluronan biosynthesis by 4-methylumbelliferone. Journal of Biological Chemistry 279(32):33281-9). It is a commercially available drug approved for use in humans as a cholagogue in several countries called "hymecromone" (Kultti A, et al. (2009) 4-Methylumbelliferone inhibits hyaluronan synthesis by depletion of cellular UDP-glucuronic acid and downregulation of hyaluronan synthase 2 and 3. Experimental Cell Research 315(11):1914-23). While it has a long and relatively reassuring safety record, questions remain about the high dosage of hymecromone required for cancer treatment.

Glycosylation is an interesting strategy to improve drugs' selective cells/tissue targeting. Due to the wide spectrum of cell receptors and the specific interactions to which carbohydrates can be specifically recognized (Molejón MI, Weiz G, Breccia JD & Vaccaro MI. (2020) Glycoconjugation: An approach to cancer therapeutics. World Journal of Clinical Oncology, 11: 110-120; Chen, F., & Huang, G. (2019) Application of glycosylation in targeted drug delivery. European journal of medicinal chemistry, 182, 111612; Cai, L., Gu, Z., Zhong, J., Wen, D., Chen, G., He, L., ... & Gu, Z. (2018) Advances in glycosylation-mediated cancer-targeted drug delivery. Drug Discovery Today, 23(5), 1126-1138). Given the specific carbohydrate-receptor interactions, sugar moieties such as glucose, galactose, mannose and rhamnose have been widely applied as specific ligands for drug administration. For example, α-glucosidase and β-galactosidase have been used to synthesize nucleosides (Binder WH, Kählig H, Schmid W. (1995) Galactosylation by use of β- galactosidase: enzymatic syntheses of disaccharide nucleosides. Tetrahedron: Asymmetry 6:1703-1710; Vankayala SL, Hargis JC, Woodcock HL. (2012) Unlocking the binding and reaction mechanism of hydroxyurea substrates as biological nitric oxide donors. J Chem Inf Model 52:1288-1297) and nucleoside-glycosylated derivatives, including the chemotherapeutic agents Ara C (Suzuki Y, Uchida K. (1994) Formation of beta-galactosyl compounds of arabinosylcytosine in growing culture of Sporobolomyces singularis. Biosci Biotechnol Biochem 58:639-643), 5-bromouridine (Uchida K, Suzuki Y. (2003) Formation of 3'-O-galactosyl compounds of 5-bromouridine by Sporobolomyces singularis. Biosci Biotechnol Biochem 67:643-645), 5-fluorouridine, 3'-azido-3'-desoxythymidine (Andreotti G, Trincone A, Giordano A. (2007) Convenient synthesis of β-galactosyl nucleosides using the marine β-galactosidase from Aplysia fasciata. J Mol Catal B Enzym 47:28-32), pyrimidine nucleoside derivatives (Yan LQ, Li N, Zong MH. (2013) First and facile enzymatic synthesis of 6-fucosyl-containing disaccharide nucleosides through β-galactosidase-catalyzed regioselective glycosylation. J Biotechnol 164:371-375) also used for analysis of acyclic nucleosides (Yan LQ, Li N, Zong MH. (2014) First enzymatic galactosylation of acyclic nucleoside drugs by β-galactosidase: Synthesis of water-soluble β-D-galactosidic prodrugs. Biotechnol Bioprocess Eng 19:586- 591) for producing potential pharmaceutical agents.

Regarding L-rhamnose, this monosaccharide is synthesized in plants, fungi and prokaryotes. However, neither L-rhamnose nor the genes responsible for its synthesis have been described in mammalians. Moreover, the presence of rhamnosidases in humans has not been described. Thus, rhamnosylated medicines may not be deglycosylated by endogenous enzymes, which guarantees the active medicines are specifically released at the sites where rhamnose receptors are located (Giang I, Boland EL, Poon GMK. (2014) Prodrug applications for targeted cancer therapy. AAPS J 16:899-913). L-rhamnose catabolism in mammals is performed through an enzyme called L-fucokinase, which catalyzes the first step of the Guanosine diphosphate fucose (GDP-fucose) and this species is incorporated into the glycosides. It was previously suggested that the L-rhamnose monomer is involved in the fucosylation process by interfering with tumor cells viability. Recent works have demonstrated that tumor cells have a lower mitotic activity in tissue slices from mice carrying a solid form of Ehrlich carcinoma. It was also shown that survival of mice was significantly improved after high doses of intraperitoneal administration of the single monomer L-rhamnose (Tomsik, Soukup, Cermakova, Micuda, Niang, Sucha, Rezacova. (2011) L-rhamnose and L-fucose suppress cancer growth in mice. Cent Eur J Biol 6: 1-9). Some studies have experimentally demonstrated the existence of specific rhamnose receptors, called RBL (rhamnose binding lectin), whose abundance is higher in cancer cells as opposed to normal noncancerous cells, suggesting the potential use of rhamnose as a new ligand for the targeted delivery of drugs in cancer therapy. An illustrative example found in nature is constituted by the plant-derived glycoalkaloids solasodine-rhamnosides, including solamargine, solasonine, and others, which induced anticancer effects in cell culture, animals, and humans. More specifically, it was shown that they induced apoptosis in cancer cells but not normal cells, due to the rhamnose-lectin interaction (Wang Y, Gao J, Gu G, Li G, Cui C, Sun B, Lou H. (2011) In situ RBL receptor visualization and its mediated anticancer activity for solasodine rhamnosides. Chembiochem 12: 2418-2420; Cham, B. E. (2017). Solasodine, solamargine and mixtures of solasodine rhamnosides: pathway to expansive clinical anticancer therapies. International Journal of Clinical Medicine, 8(12), 692-713.). However, to the best of the present inventors' knowledge, the technological use of rhamnosylation as a strategy for cancer-targeting has been only successful when combined with the hydrolysis of the Rha-conjugate in the target organ, using the so-called lectin-directed enzyme-activated prodrug therapy (LEAPT) bipartite drug delivery system (Robinson MA, Charlton ST, Garnier P, Wang XT, Davis SS, Perkins AC, Frier M, Duncan R, Savage TJ, Wyatt DA, Watson SA, Davis BG. (2004) LEAPT: lectin-directed enzyme-activated prodrug therapy. Proc Natl Acad Sci U S 101: 14527-14532; Garnier P, Wang XT, Robinson MA, van Kasteren S, Perkins AC, Frier M, Fairbanks AJ, Davis BG. (2010) Lectin-directed enzyme activated prodrug therapy (LEAPT): Synthesis and evaluation of rhamnose-capped prodrugs. J Drug Target 18: 794-802; Ma Y, Chen H, Su S, Wang T, Zhang C, Fida G, Cui S, Zhao J, Gu Y. (2015) Galactose as Broad Ligand for Multiple Tumor Imaging and Therapy. J Cancer 6: 658-670). That is, on one side, Rha-capped prodrugs were chemically synthesized. On the other side, an artificially modified rhamnosidase with terminal galactose at the N-linked carbohydrate chains (galactosylated rhamnosidase) was employed to specifically target the asialoglycoprotein receptor (ASGPR). The ASGPR protein is a membrane-bound, endocytic lectin found in abundance on the surface of hepatocytes in the liver. Moreover, ASGPRs have the ability to trigger receptor-mediated endocytosis (RME) and transfer their ligands inside the cell. Using this system, both the Rha-capped prodrug and the specific enzyme were delivered to the liver as a strategy for treatment against HCC. These two steps were effectively combined *in vivo* (trials using human hepatocellular liver carcinoma cell line HepG2 in nude athymic mice), with activation of the prodrug resulting in *in site* selective release of the parent drug (Robinson MA, Charlton ST, Garnier P, Wang XT, Davis SS, Perkins AC, Frier M, Duncan R, Savage TJ, Wyatt DA, Watson SA, Davis BG. (2004) LEAPT: lectin-directed enzyme-activated prodrug therapy. Proc Natl Acad Sci U S 101: 14527-14532; Garnier P, Wang XT, Robinson MA, van Kasteren S, Perkins AC, Frier M, Fairbanks AJ, Davis BG. (2010) Lectin-directed enzyme activated prodrug therapy (LEAPT): Synthesis and evaluation of rhamnose-capped prodrugs. J Drug Target 18: 794-802; Ma Y, Chen H, Su S, Wang T, Zhang C, Fida G, Cui S, Zhao J, Gu Y. (2015) Galactose as Broad Ligand for Multiple Tumor Imaging and Therapy. J Cancer 6: 658-670). As stated by the authors of the mentioned works, the rhamnose cap would serve (1) to decrease the cytotoxicity of the drug before its release, (2) to deliver the prodrug to specific cell types -in this case hepatocytes after binding to ASGPR and subsequent endocytosis, and (3) to increase the sometimes poor solubility of drugs used in certain therapies (Garnier P, Wang XT, Robinson MA, van Kasteren S, Perkins AC, Frier M, Fairbanks AJ, Davis BG. (2010) Lectin-directed enzyme activated prodrug therapy (LEAPT): Synthesis and evaluation of rhamnose-capped prodrugs. J Drug Target 18: 794-802). Further, the work by Xu et al., (2019) demonstrated that Rha-capped fluorescent dye Rhodamine B (Rha-RhB) had a higher cell affinity and cellular internalization rate of oral cancer cell KB and breast cancer cell MDA-MB-231 than that of the normal epithelial cells MCF 10A. However, the rhamnosylated anticancer drugs resulted in little cytotoxic effects on cancer cells. Although the restoration of the cytotoxic activity could be demonstrated *in vitro* by addition of a α-rhamnosidase, the *in vivo* application of such system has not been demonstrated (Xu L, Liu X, Li Y, Yin Z, Jin L, Lu L, Qu J, Xiao M. (2019) Enzymatic rhamnosylation of anticancer drugs by an α-L-rhamnosidase from Alternaria sp. L1 for cancer-targeting and enzyme-activated prodrug therapy. Appl Microbiol Biotechnol 103: 7997-8008). In conclusion, the use of Rha-capped drugs would only be possible in combination with a lectin-directed enzyme (LEAPT) or provided drugs are found where the cytotoxicity of the parental compound is not substantially decreased by the glycosylation. The examples listed above refer to rha-capped prodrugs, whose activity was lower than the activity of the parental drug. It is nearly impossible to define general patterns of biological activities of the glycosides comparable to the those of respective aglycones (Kren V. 2001. Chemical Biology and Biomedicine of Glycosylated Natural Compounds. In: Fraser-Reid B.O., Tatsuta K., Thiem J. (eds) Glycoscience: Chemistry and Chemical Biology I-III. Springer, Berlin, Heidelberg.). First, glycosylation modifies the structure of drugs, which can prevent the receptors of that drug from recognizing it, decreasing (or completely suppressing) its activity. Additionally, it can also be recognized by other receptors, changing its activity, or generating side effects. Secondly, glycosylation generally increases the solubility of the respective aglycone, which can translate into greater bioavailability, or on the contrary, faster elimination from the bloodstream with a consequent lower concentration in the target organ. Even more, the comparison of the activity of diglycosylated drugs cannot find any straightforward comparison with mono-glycosylated drugs. Several examples were reported where disaccharides have a different performance regarding the monosaccharides. For instance, the binding enthalpies of certain lectins to disaccharides were about twice the binding enthalpies of the monosaccharides (Surolia, A., Sharon, N., & Schwarz, F. P. (1996). Thermodynamics of monosaccharide and disaccharide binding to Erythrina corallodendron lectin. Journal of Biological Chemistry, 271(30), 17697-17703). Other example is the fucosyltransferase inhibitory activity of the disaccharide (α-D-manopyranose-1,3-GaINAc), that reduces fucose incorporation onto the surface carbohydrates of malignant cells, leading to a lower invasive capacity. This inhibitory activity was not detected with any of the single sugar moieties (N-acetyl-galactosamine and D-mannose) (Vogel, P. (2001). Monosaccharide and disaccharide mimics: new molecular tools for biology and medicine. CHIMIA International Journal for Chemistry, 55(4), 359-365).

The present inventors have shown that the use of the coumarin 4-methylumbelliferone (4MU) in cell lines of HCC and other tumors, has an antifibrotic effect, inhibits the development of satellite nodules and enhances the development of an immune response against the tumor (Piccioni F, Malvicini M, Garcia MG, Rodriguez A, Atorrasagasti C, Kippes N, Piedra Buena IT, Rizzo MM, Bayo J, Aquino J, Viola M, Passi A, Alaniz L, Mazzolini G. (2012) Antitumor effects of hyaluronic acid inhibitor 4-methylumbelliferone in an orthotopic hepatocellular carcinoma model in mice. Glycobiology 22:400-410; Malvicini M, Fiore E, Ghiaccio V, Piccioni F, Rizzo M, Olmedo Bonadeo L, Garcia M, Rodriguez M, Bayo J, Peixoto E, Atorrasagasti C, Alaniz L, Aquino J, Matar P, Mazzolini G. (2015) Tumor Microenvironment Remodeling by 4-Methylumbelliferone Boosts the Antitumor Effect of Combined Immunotherapy in Murine Colorectal Carcinoma. Mol Ther 23:1444-55; Rodriguez MM, Fiore E, Bayo J, Atorrasagasti C, Garcia M, Onorato A, Domínguez L, Malvicini M, Mazzolini G. (2018) 4Mu Decreases CD47 Expression on Hepatic Cancer Stem Cells and Primes a Potent Antitumor T Cell Response Induced by Interleukin-12. Mol Ther 26:2738-2750).

Herein a glycosylated derivative of 4MU is presented, namely rutinosyl-4-methylumbelliferone (4MUR). This compound carries the disaccharidic rutinosyl unit (6-O-α-L-rhamnosyl-β-D-glucose) covalently bound to 4MU. Unlike the previously reported rha-capped drugs, 4MUR shows significantly higher antitumoral effects than those obtained with the parental compound 4MU. As will be disclosed herein, the present inventors have now shown that 4MUR presents greater efficacy when compared to 4MU through *in vitro* tests and an orthotopic model (designed by the inventors) of HCC associated with fibrosis.

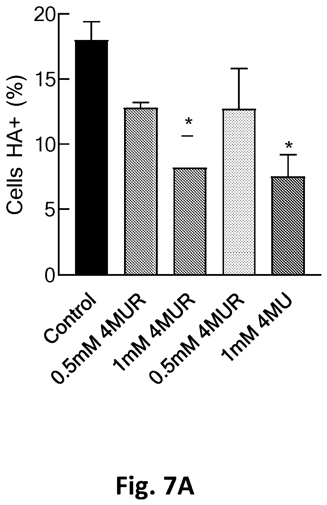

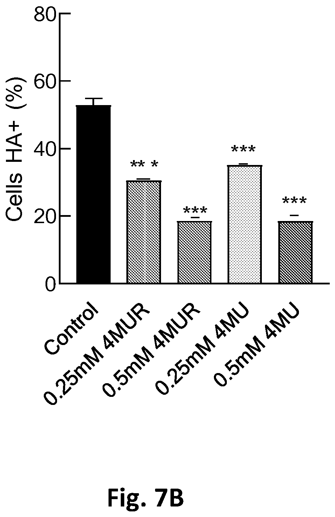

Briefly, according to the tests carried out by the present inventors:
- The *in vitro* antitumoral effect of 4-MUR against hepatocarcinoma was significantly higher than 4-MU, in murine (Hepa 1.6) as well as human (Huh7) cells.
- Cell Viability of hepatic stellate (CFSC-2G) and non-tumoral hepatic cells (IHH) showed lower toxicity to 4MUR treatment than to 4MU.
- 4-MUR showed higher reduction of cellular proliferation and induction of apoptosis on tumoral cells Hepa129 in comparison with 4-MU.
- In Huh7 cells, the expression of HAS2 and HAS3 was significantly reduced upon 4MUR treatment (p<0.05), while 4MU treatment did not affect the expression of HAS2 but up-regulated HAS3. In the CFSC-2G hepatic stellate cell line, 4MUR did not affect HAS2 expression, but decreased HAS3 expression. Whereas 4MU upregulated HAS2 and HAS3 expression.
- HA levels were significantly decreased under 4MUR and 4MU treatments in CFSC-2G and Hepa129 cells (Fig. 7).
- In CFSC-2G the fibrotic related genes α-SMA, COL1A2, TGF-β and TIMP-1 were strongly downregulated, and the antifibrogenic gene matrix metalloproteinase, MMP-2 was not affected after 4MUR treatment. These results, indicate that 4MUR induced a reduction of CFSC-2G activation and consequently reduced the fibrosis degree.
- Regarding in vivo studies 4MUR treatment showed that: mice tumor volume was significantly reduced, histological analysis of organs (spleen, heart, and lung) showed no necrotic areas, there were no significant changes noticed for animal body weight, the amount of ascitic fluid was significantly reduced and animal survival was increased.
- Mice treated with 4MUR revealed a significant reduction in the fibrosis degree measured by collagen deposits and HA content.

The derivative compound of the invention has no relationship, in terms of its chemical structure, with currently approved antitumor compounds. However, as shown herein, the results obtained by the present inventors prove that this drug is useful for the treatment of HCC and advanced HCC.

Even when the structure and synthesis of this glycoconjugated drug was previously disclosed (Mazzaferro LS, Piñuel L, Erra-Balsells R, Giudicessi SL, Breccia JD. (2012) Transglycosylation specificity of Acremonium sp. α-rhamnosyl-β-glucosidase and its application to the synthesis of the new fluorogenic substrate 4-methylumbelliferyl-rutinoside. Carbohydr Res 347: 69-75), its biological activity was not tested previously. Moreover, it has been found by the present inventors that this glycosylated coumarin derivative is not only effective against tumor proliferation in hepatocarcinoma, but also that its efficiency is remarkably higher than the unglycosylated form. Therefore, the present invention provides a new alternative to currently used therapies for this particular cancer, by disclosing the use of a coumarin derivative for the treatment of hepatocarcinoma.

The glycoside hydrolases (GHs) are enzymes that hydrolyze the glycosidic bond between two carbohydrates or between a carbohydrate and a residue of another nature. Additionally, these catalyzers have the ability to form glycosidic bonds (Bissaro B, Monsan P, Fauré R, O'Donohue MJ. (2015) Glycosynthesis in a waterworld: new insight into the molecular basis of transglycosylation in retaining glycoside hydrolases. Biochem J 467:17-35; Cobucci-Ponzano B, Moracci M. (2012) Glycosynthases as tools for the production of glycan analogs of natural products. Nat Prod Rep 29: 697-709; Planas A & Faijes M. (2002) Glycosidases and Glycosynthases in enzymatic synthesis of oligosaccharides. An overview. Afinidad Barcelona 59: 295-313). Therefore, GHs are able to synthesize glycoconjugates, the syntheses can be performed *in vitro* by reverse hydrolysis (thermodynamic control) or transglycosylation (kinetic control) (Watts AG, Withers SG. (2004) Glycosynthase-catalysed formation of modified polysaccharide microstructures. J Biotechnol 380: 9-10). Diglycosidases are GHs that recognize and hydrolyze diglycoconjugates at the heterosidic bond, releasing disaccharides and the corresponding aglycone. Those that have a retaining mechanism are capable of transglycosylating this disaccharide into an acceptor molecule (Mazzaferro L, Piñuel L, Minig M, Breccia JD. (2010). Extracellular monoenzyme deglycosylation system of 7-O-linked flavonoid β-rutinosides and its disaccharide transglycosylation activity from Stilbella fimetaria. Archives of Microbiology, Volume 192, pages 383-393(2010); Erratum: Archives of Microbiology, Volume 193, page 461 (2011)).

The present inventors have described two fungal diglycosidases (α-rhamnosyl-β-glucosidase I and II) from an *Acremonium* sp. strain. These fungal enzymes have the ability to transfer the disaccharide rutinose to different acceptors (Mazzaferro L, Piñuel L., Minig M, Breccia JD. 2010. Extracellular monoenzyme deglycosylation system of 7-O-linked flavonoid β-rutinosides and its disaccharide transglycosylation activity from Stilbella fimetaria. Archives of Microbiology 192:383-393*.* Erratum: Archives of Microbiology (2011) 193: 461*;* Weiz G., Mazzaferro LS, Kotik M, Neher BD, Křen V, Breccia JD. 2019. The flavonoid degrading fungusř Acremonium sp. DSM 24697 produces two diglycosidases with different specificities. Applied Microbiology and Biotechnology 103: 9493-9504). The present inventors have synthesized this derivative molecule using α-rhamnosyl-β-glucosidase I. Other diglycosidases obtained from fungi were reported in the last years by other groups and could also be used for this purpouse (Šimčíková D, Kotik M, Weignerová L, Halada P, Pelantová H, Adamcová K, Křen V. (2015) α-L-Rhamnosyl-β-D-glucosidase (rutinosidase) from Aspergillus niger: characterization and synthetic potential of a novel diglycosidase. Adv Synth Catal 356:1-12; Ishikawa M, Kawasaki M, Shiono Y, Koseki T. (2018) A novel Aspergillus oryzae diglycosidase that hydrolyzes 6-O-α-L-rhamnosyl-β-D-glucoside from flavonoids. Appl Microbiol Biotechnol 102:3193-3201).

Therefore, the present invention provides useful alternatives to currently available therapies for hepatocarcinoma.

### SUMMARY OF THE INVENTION

According to a first object, the present invention provides a composition comprising a diglycosylated coumarin derivative, in particular a rutinosylated coumarin derivative, which is rutinosyl-4-methylumbelliferone, also referred to herein as 4-methylumbelliferyl-rutinoside, or 4MUR for the treatment of hepatocarcinoma (HCC).

According to another object, the present invention describes reducing tumor cell proliferation in hepatocarcinoma (HCC) comprising administration of 4-methylumbelliferyl-rutinoside (4MUR) or a composition comprising the same.

### BRIEF DESCRIPTION OF THE DRAWINGS

The following figures form part of the present specification and are included to further illustrate certain aspects of the present invention.
**Figure 1****:** Reaction scheme for the enzymatic synthesis of 4-methylumbelliferyl-rutinoside (4MUR).
**Figure 2****:** Viability of hepatocellular tumoral cells: **(2A)** Huh7 (human hepatic cell line), **(2B)** Hepa1-6 (murine hepatic cell line) treated with conjugate compound 4MUR y parental compound MU.
**Figure 3****:** Cell viability of hepatic non-tumoral cells: **3A** CFSC-2G (Rat hepatic stellate cell line) **(3B)** IHH (human hepatocytes cell line).
**Figure 4****:** *In vitro* proliferation of Hepa129 cells treated with different concentrations of either 4MU or 4MUR, as determined by ³H-thymidine incorporation. RPMI medium was used as a control. ***P < 0.01 vs control. Results are expressed as proliferation mean ± SEM.
**Figure 5****:** Evaluation of apoptosis in Hepa129 cells treated with 4MU or 4MUR, through morphological features assessment visualized by acridine orange and ethidium bromide staining. Bars represent average viable cells (grey bars) or apoptotic cells (black bars) detected in each group.
**Figure 6****:** Hyaluronic acid inhibition by 4MUR and 4MU treatment. Relative mRNA expressions of hyaluronan synthases HAS2 and HAS3 after treatment of 0.5 and 1.0 mM of 4MU and 4MUR. mRNA expression was normalized to reference genes 18S and ACTB. Statistical analysis: *p < 0.05, **p < 0.01 compared to untreated control of each cell line (0.0 mM = 1.0).
**Figure 7****:** (7A) CFSC-2G and (7B) Hepa 129 cells were treated with different concentrations (0-1 mM) 4MUR or 4MU for 48 h. HA content was analyzed by flow cytometry. * p<0.05; ** p<0.01 ***p<0.001 vs Control by One-way ANOVA and Tukey post-test.
**Figure 8****:** Antifibrotic effect on hepatic stellate cell line (CFSC-2G) in vitro treated with 4MUR or 4MU. mRNA levels of cell activation and liver fibrosis-associated genes α-SMA, COL1A2, TGF-β1, TIMP-1, and MMP-2 were analyzed. Bars represent the average ±SEM of expression fold changes on CFSC-G2 cells treated with DMEM (control) 4MUR or 4MU for 48 h * p<0.05; ***p<0.001 vs Control by One-way ANOVA and Tukey post-test.
**Figure 9****: (9A)** Tumor volume (mm³) of fibrotic mice administered with 4MU, 4MUR or control (saline solution), at day 20 post-Hepa129 inoculation. **(9B)** Photographs of intrahepatic tumors treated with saline, 4MU and 4MUR. Left panel, image of the tumor on day 5. Right panel: magnified image of the tumor on day 20.
**Figure 10****:** Representative microphotographs of liver sections stained with Sirius red (20X magnification) and collagen quantification by morphometric analysis and densitometry. *p<0.05; **p < 0.01 Control vs 4MUR or 4MU, One-way ANOVA and Tukey post-test. Bars represent the average of measures of each group ± SEM.
**Figure 11****:** Liver tissue HA expression: microphotography of hepatic HA staining by HA-bP (10x, 20x). Arrows indicate HA deposits (seen in black). Scale bar = 50 µm. Quantification of HA-positive sections by densitometry, *p<0.05; **p < 0.01. Bars represent the average of measures of each group ± SEM
**Figure 12****: (12A)** Animals weight curve during experimental assay. **(12B)** Hematoxylin/eosin staining of different tissues (liver, heart, spleen, lung, and kidney) from mice treated with either 4MU or 4MUR. **(12C):** Survival of mice subjected to treatment with either 4MU or 4MUR. Log-Rank test; percentage of animal survival on Day 20: with Saline (negative control) 75%; 4MU treatment 66%; 4MUR treatment 100%.

### DETAILED DESCRIPTION OF THE INVENTION

The present invention is based on a structural modification carried out on a coumarin derivative (also referred to herein as parental drug), a drug currently approved for its spasmolytic properties, and also used as a choleretic and light-protective agent. As previously mentioned herein, this parental drug, called 4-methylumbelliferone (4MU), has been experimentally used and shown to have antitumor effect against HCC.

The present inventors have now demonstrated that a diglycosylated derivative of 4MU presents substantially higher antitumoral effects when compared to 4MU, thus providing an interesting alternative to current therapies for hepatocellular carcinoma.

The present inventors have modified this parental drug (4-methylumbelliferone) by adding a rutinosyl unit (6-O-α-L-rhamnopyranosyl-β-D-glucopyranose) through a reaction catalyzed by the wildtype and/or the recombinant enzyme called 6-O-α-rhamnosyl-β-glucosidase from *Acremonium* sp. DSM24697 (Mazzaferro LS, Piñuel L, Erra-Balsells R, Giudicessi SL, Breccia JD. (2012) Transglycosylation specificity of Acremonium sp. α-rhamnosyl-β-glucosidase and its application to the synthesis of the new fluorogenic substrate 4-methylumbelliferyl-rutinoside. Carbohydr Res 347: 69-75). Said enzyme is a diglycosidase previously disclosed by CONICET and Universidad Nacional de La Pampa (Breccia et al., AR Patent Application Number 20120101056).

### Preparation example - Synthesis of 4-methylumbelliferyl-rutinoside

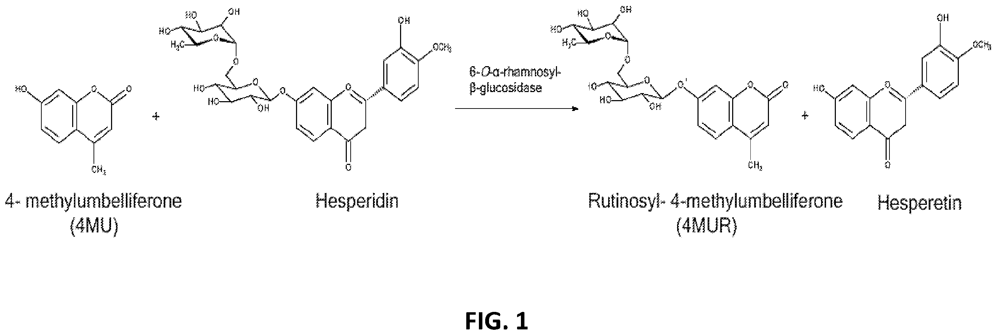

The compound 4-methylumbelliferyl-rutinoside (4-MUR) was synthesized using the recombinant enzyme 6-O-α-rhamnosyl-β-glucosidase (EC 3.2.1.168), hesperidin as the glycosidic (rutinose) donor and 4-methylumbelliferone as acceptor. A schematic diagram of the synthesis is illustrated in Fig. 1. The reaction conditions were as follows: pH 5.0, 30°C. The reagents used were hesperidin 1.8 mM, 4-methylumbelliferone 1.8 mM (1:1 ratio) and DMSO 2% (v/v). A higher yield in 4MUR (33% with respect to 4- methylumbelliferone) was obtained in a 15 min-process.

As can be seen from the reaction scheme shown in Fig. 1, the conjugated compound described herein, 4-methylumbelliferyl-rutinoside, differs from 4-methylumbelliferyl-glucoside in the rhamnosyl substitution at the C-6 of glucose.

### Pharmaceutical compositions

The rutinosylated coumarin derivatives disclosed herein, such as 4-methylumbelliferyl-rutinoside, may be formulated into pharmaceutical compositions, together with an acceptable excipient, diluent or carrier, for administration through oral, parenteral, intraperitoneal, subcutaneous or intravenous routes.

According to a preferred embodiment, the rutinosylated coumarin derivatives are comprised in a pharmaceutical composition in liquid form. Alternatively, rutinosylated coumarin derivatives may be freeze-dried ready for reconstitution with a suitable diluent prior to administration. Since, the glycosylated derivative is water soluble, various buffer diluent or water could be suitable for administration.

The pharmaceutical compositions according to the present invention generally comprise a therapeutically effective amount of the rutinosylated coumarin derivatives. As proven herein, 4-MUR was efficient against hepatocellular carcinoma when it was administered i.p. (20 mg/kg/day) as well as through oral administration (200 mg/kg/day).

### EXAMPLES

The present invention is further illustrated by the following Examples, which are not intended to limit the scope thereof. Instead, the Examples set forth below should be understood only as exemplary embodiments for better taking into practice the present invention.

### Example 1 - Antitumor activity of 4MUR in vitro

In order to evaluate the therapeutic efficacy and potential cytotoxicity of the glycoconjugated drug of the invention for the treatment of HCC, cell viability assays were carried out in different cell lines.

To this end, an *in vitro* model was designed. The cells used for *in vitro* assay were a murine hepatic cell line (Hepa1-6) and a human hepatic cell line (Huh7).

Chemiograms were performed using increasing doses of the compound 4-MU as well as the glycosylated compound 4MUR (0-2000 µM). Cell viability was measured by using the CellTiter kit (Promega Corporation). Proliferation was measured through a tritiated thymidine incorporation assay, by incubating the cells for 48 h with either one of the compounds (4MU/4MUR) or culture medium (RPMI) in the presence of ³H-thymidine (Migliore-LaClaustra).

After incubation, cells were harvested and centrifuged to remove ³H-thymidine from the supernatant. The cell pellet was lysed with sterile distilled water (200 µl) and said volume was added to paper filters for retaining cell DNA with incorporated ³H-thymidine. Each paper filter was impregnated in a tube containing scintillation liquid and each sample was run and read using a liquid scintillator counter (Beckman) detecting deviations per minute emitted by tritium (³H). Results are expressed as counts per minute (cpm) for each sample.

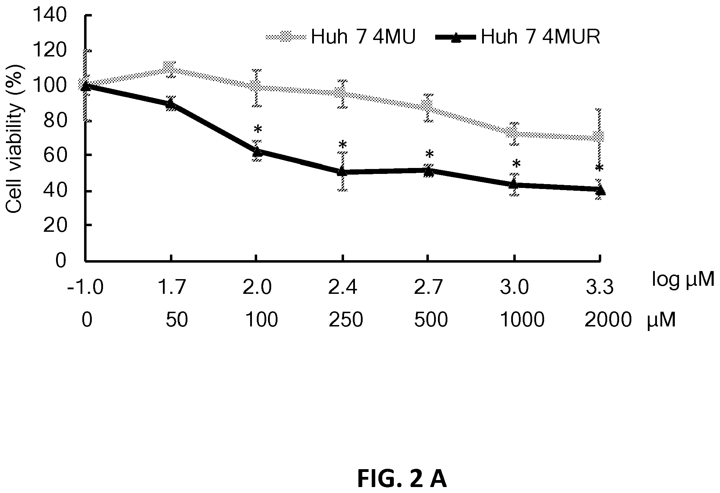

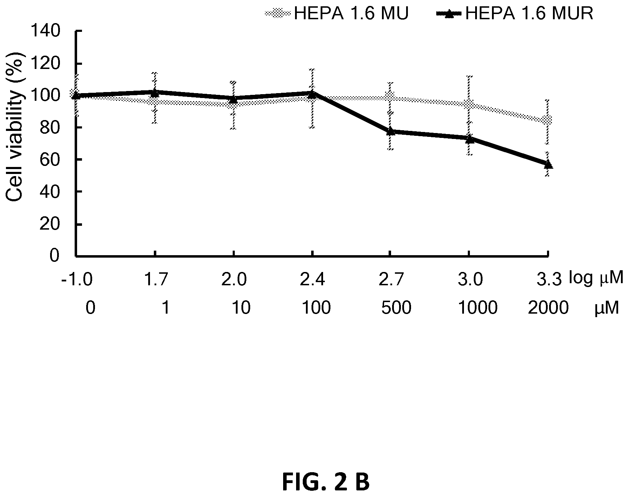

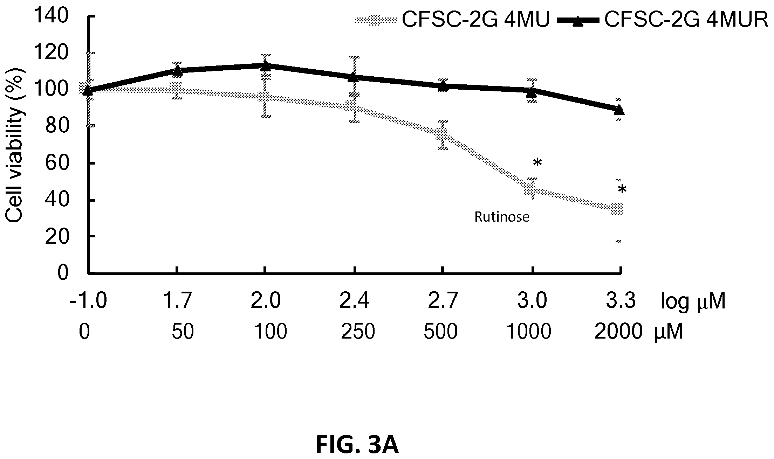

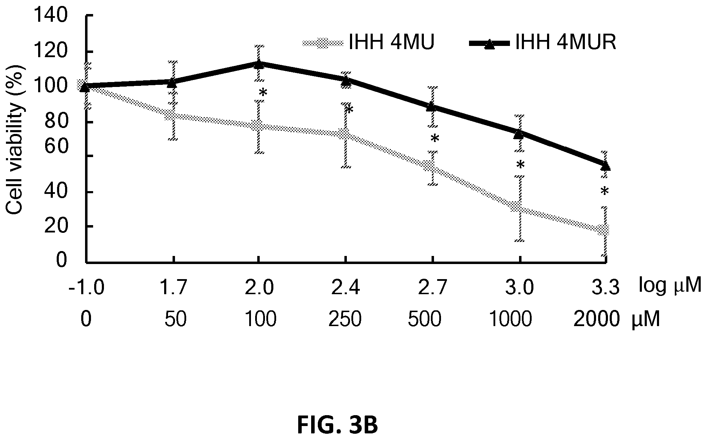

4MUR has a significant anti-proliferative effect on liver tumoral cells, in a concentration-dependent manner (p<0.05). When we compared the activity of 4MUR and 4MU, the results showed that glycosylation increased 4MU antitumoral effect by 2.2 on Huh7 (Fig. 2A -2B; Table 1). Importantly, the half-inhibitory concentration (IC₅₀) values of 4MUR in non-tumoral cells CFSC-2G and IHH were higher than those of tumor cells (Table 1). Compared to 4MU, IC₅₀ values of 4MUR increased 6.2-fold and 25.8-fold for IHH and CFSC-2G cell lines, respectively (Fig. 3A-3B, Table 1). This data showed that the rutinosylated compound, target specifically liver tumoral cells suggesting potential cancer selectivity of 4MUR.

**Table 1: 4MU and 4MUR IC₅₀ values for different cell lines**

| **Cell lines** | **IC₅₀ values (µM)** | |
|---|---|---|
| | **4MU** | **4MUR** |
| Huh7 | >2000 | 488 |
| CFSC-2G | 1161 | >2000 |
| IHH | 486 | >2000 |

### Example 2 - Cell proliferation and induced apoptosis in Hepa129 cells treated with 4MUR in vitro

Proliferation capacity of murine Hepa129 cells treated with 4MU and 4MUR was evaluated, as measured by ³H-thymidine incorporation.

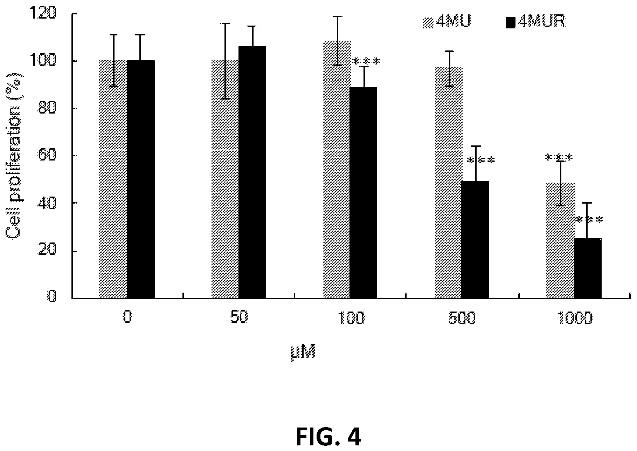

As illustrated in Fig. 4, treatment with 4MUR significantly decreased proliferation capacity at low doses (0.1 mM), while treatment with 4MU (parental drug) required a dose of 1 mM for difference in proliferation to be detected. In other words, 4MUR significantly decreased proliferation at a 10-times lower dose (0.1 mM) (Fig. 4). The results obtained with the higher dose (1 mM) show a 50% lowered proliferation of cells treated with 4MU, while those cells treated with 4MUR did not proliferate.

On the other hand, it was examined if growth inhibition by 4MUR was due to induction of apoptosis. To this end, cells were stained with an orange solution of acridine-ethidium bromide (Acridine Orange (NA) / Ethidium Bromide (BrEt) staining) and were then visualized by fluorescence microscopy to analyze their morphological characteristics.

Briefly, after 48h of treatment, the cells were incubated with a mixture of NA/BrEt (1ug/ml) and were then observed under a fluorescence microscope (Nikon). Viable cells are permeable to NA and are stained green. Cells with an altered plasma membrane allow BrEt to enter, which displaces NA, staining the cell orange. The percentage of viable or apoptotic cells was counted for each treatment.

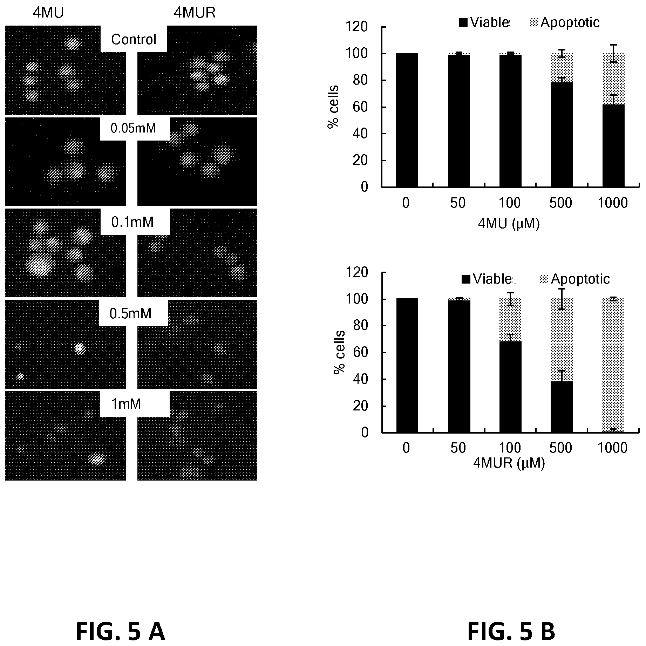

4MUR significantly induced apoptosis in Hepa129 cells at a dose of 0.1 mM, whereas cells treated with 4MU at this concentration were resistant (Fig. 5).

At the highest dose concentration (1 mM) tested, 60% of 4MU-treated cells presented death by apoptosis. It is worth noting that 100% of cells treated with this concentration of 4MUR showed apoptosis.

### Example 3 - Expression levels of ASGP receptors in Huh, CFSC-2G and IHH cell lines

The major role of ASGP receptors is the binding and the internalization of glycoconjugates that contain terminal galactose, rhamnose, or N-acetylgalactosamine residues ( D'Souza AA, Devarajan P V. Asialoglycoprotein receptor mediated hepatocyte targeting - Strategies and applications. Vol. 203, Journal of Controlled Release. Elsevier; 2015. p. 126-39.). ASGPR is expressed almost exclusively in liver cells with higher expression in tumoral cell lines (Cavallaro G, Farra R, Craparo EF, Sardo C, Porsio B, Giammona G, et al. Galactosylated polyaspartamide copolymers for siRNA targeted delivery to hepatocellular carcinoma cells. International Journal of Pharmaceutics. 2017;525(2):397-406). These results were confirmed by measuring mRNA expressions using qPCR of ASPGR 1 and ASPGR 2 in Huh7 and IHH. In addition, the CFSC-2G hepatic stellate cell line showed lower ASGPR normalized expression than all the tumoral cells analyzed (Table 2).

**Table 2. ASGP receptors expression in different cell lines**

| **Cell lines** | ***ASGP-R 1 mRNA amount*** | |
|---|---|---|
| | ***normalized to βActin RNA*** | |
| | **ASGPR 1** | **ASGPR2** |
| JHH6 | 0.075 ± 0.004 | 0.215± 0.032 |
| Huh7 | 1.485 ± 0.177 | 1.964 ± 0.389 |
| IHH | 0.025 ± 0.003 | 0.071± 0.015 |
| CFSC-2G | 0. 032 ± 0.003 | 0.026 ± 0.006 |
| HEPA1.6 | 12.93 ± 2.63 | 49.20 ± 1.648 |

### Example 4 - 4MUR regulates the expression of HAS genes, HA contents and decreases hepatic stellate cells activation.

Since HAS mRNA levels often correspond to the activity of hyaluronan synthesis, the effect of 4MUR on HAS2 and HAS3 expression was assessed.

To this end, cells with a concentration of 100,000 cells/ml were cultured on 6-well plates and treated with 4MUR (500 µM) for 24 h. Total RNA was extracted using the TriReagent (Sigma Aldrich) according to the manufacture's protocol. RNA was quantified at 260 nm in a DU^{®}730 spectrophotometer (Beckman Coulter, Fullerton, CA, USA). The RNA purity was evaluated according to MIQE guidelines by measuring the ratio A260/A280.

The cDNA synthesis was obtained according to the manufacture's suggestions (Applied Biosystems, Foster City, CA, USA). RT-qPCR for HAS2, HAS3 genes was performed using iQ SYBR Green Supermix protocol (Bio-Rad Laboratories, Milan, Italy). Each PCR amplification was carried out following manufacters instructions (iQ SYBR Green Supermix-Bio-Rad) in a Bio-Rad iQ5 real-time PCR detection system (iCycler IQ5 software, version 3.1; Bio-Rad) Expression was normalized to actin with two reference genes.

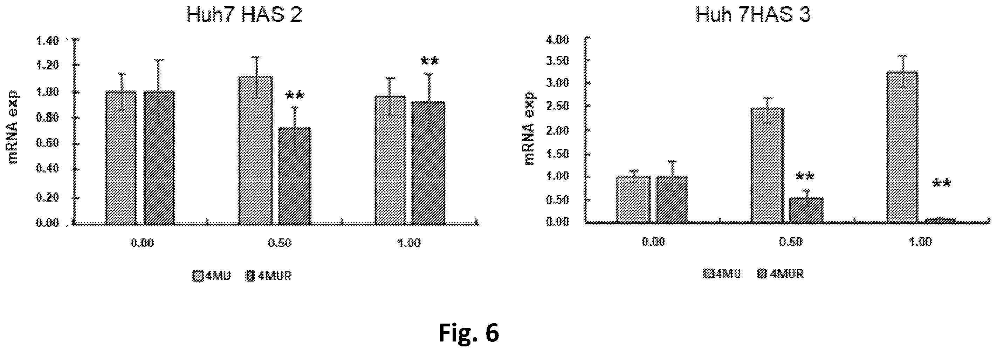

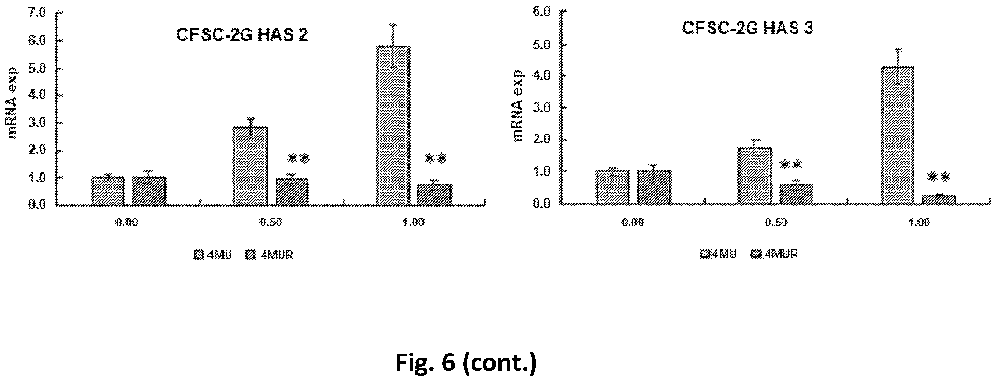

In Huh7 cells, the expression of HAS2 and HAS3 was significantly reduced upon 4MUR treatment (p<0.05) (Fig.6). On the other hand, 4MU treatment did not affect the expression of HAS2 but up-regulated HAS3 by around 3.5-fold (Fig. 6). In the CFSC-2G hepatic stellate cell line, 4MUR did not affect HAS2 expression, where it was upregulated by 4MU (around 5-fold). Accordingly, HAS3 expression was down regulated after 4MUR treatment (4.5-fold) where it was upregulated by 4MU (4.2-fold) (Fig. 6).

The levels of HA were quantified in the activated stellate cells CFSC-2G. For this goal, HA was detected by incubation with HA-fluorescein iso-thiocyanate (FITC) (20 µg/100 µL, Calbiochem SA, Bs. As., Argentina). Cells were treated with 4MU or 4MUR 0.25-1 mM and fixed were analyzed by flow cytometry with a FACS Accuri, BD (Becton Dickinson Immunocytometry Systems), using the BD Accuri 6C^{™} software. HA level was significantly decreased by both 4MUR and 4MU, indicating a role in the activation of these cells (Fig. 7A). Additionally, the HA contents was also significantly decreased under 4MUR and 4MU treatments in the tumoral Hepa129 cells (Fig. 7B).

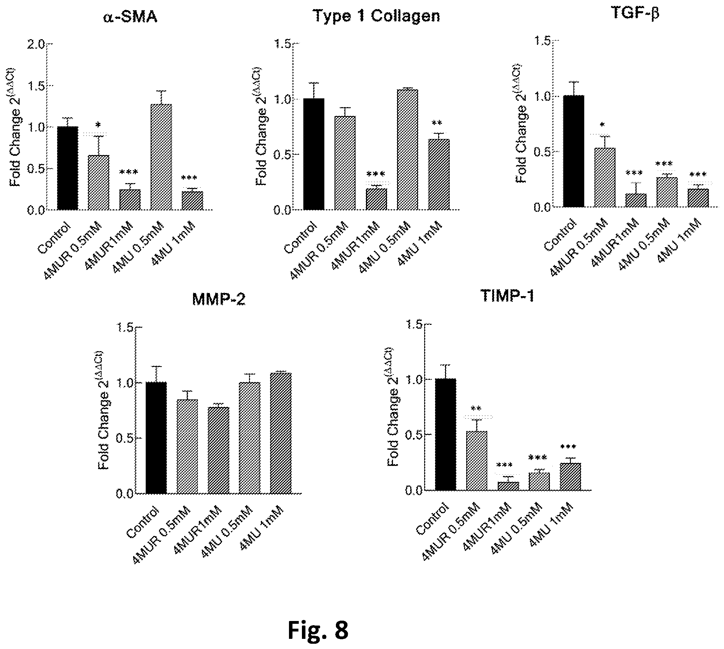

The effect of 4MUR on liver fibrogenesis was assessed by the expression of the fibrotic-related genes including α-SMA, TGF-β, profibrogenic (COL1A2 and TIMP-1), and the antifibrogenic gene matrix metalloproteinase (MMP-9) on CFSC- 2G cells by qPCR. It should be noted that while α-SMA, COL1A2, TGF-β and TIMP-1 were strongly downregulated in CFSC-2G cells, MMP-9 was not affected (Fig. 8). It indicated that 4MUR induced a reduction of CFSC-2G activation and consequently reduced the fibrosis degree.

### Example 5 - Antitumor and antifibrotic effect of 4MUR in a murine model of hepatocellular carcinoma associated to advanced fibrosis.

To assess the *in vivo* antitumoral efficacy of 4MUR, an orthotopic tumor model associated with advanced fibrosis induced by TAA was established

C3H/He mice were intraperitoneally (i.p.) treated with 20 mg/kg thioacetamide (TAA) 3 times a week, for 4 weeks, in order to induce liver fibrosis. On day 28, mice were anesthetized and underwent surgery and orthotopic tumors were established by subcapsular intrahepatic inoculation of 1.25x10⁵ Hepa129 cells into the left lobe of the liver (Day 0).

Between day 5 and day 7 post-administration of the cells, animals were again subjected to surgery for observing tumor development. Mice were divided into 3 groups (n = 5) and each group received: (1) Saline solution (control); (2) 4MU dissolved in water, at a dose of 20 mg/Kg; (3) 4MUR dissolved in water at a dose of 20 mg/Kg. Intraperitoneal administration for different groups began on day 5 and was performed 3 times a week, until the end of the experiment on day 21 post Hepa129 inoculation. On day 21, animals were sacrificed, mice weight was recorded and ascites grade (mild, moderated, severe, and hemorrhagic), as well as tumor volume (mm³), was calculated by the formula π/6× larger diameter × (smaller diameter)², by caliber measurement.

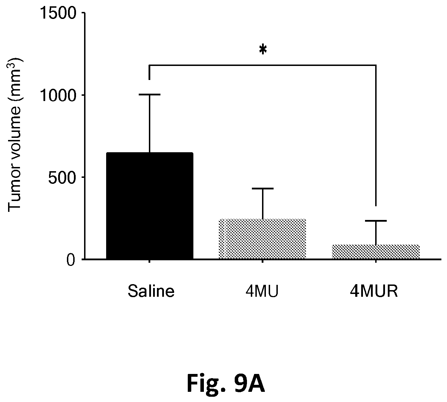

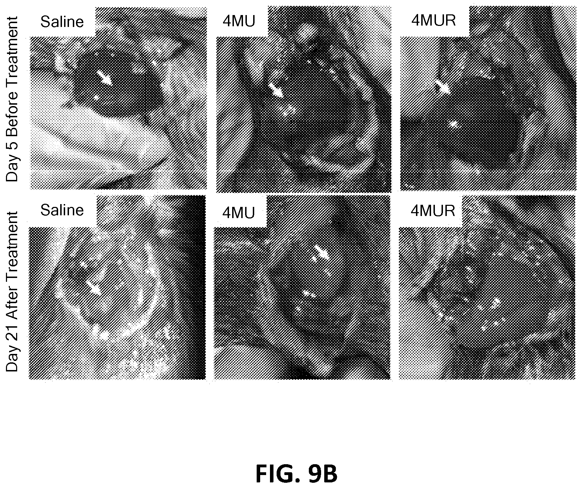

The tumor size from control (n = 5), 4MU-treated (n = 5), and 4MUR-treated (n = 5) mice were analyzed on day 21 post Hepa129 inoculation. 4MU treatment showed inhibition of HCC tumor growth in comparison with untreated mice, although these results were not statistically significant (243.8 ± 94.0 mm³ versus 650.5 ± 176.7 mm³, Fig. 9A). Notably, 4MUR treatment significantly decreased HCC tumor volume compared to control (113.5±77.8 mm³; p<0.05) (Fig. 9A and 9B). Remarkably, in 60% of the animals that received 4MUR, tumors were not detected macroscopically at the endpoint of the experiments (day 21 post-Hepa129), compared to 20% in 4MU treated-mice (Fig. 9B).

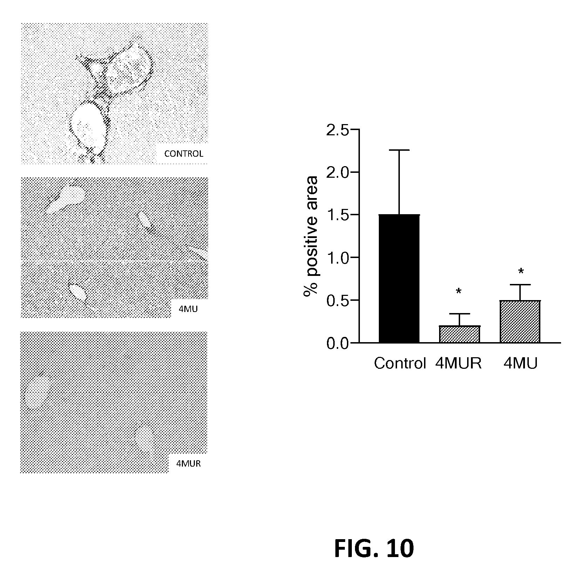

The histological analysis of mice treated with 4MUR revealed a significant reduction in the fibrosis degree measured by collagen deposits (0.53 ± 0.04 vs 0.21 ± 0.02 for 4MU and 4MUR, respectively, p<0.05) (Fig. 10).

The 4MUR treatment on liver fibrogenesis was evaluated in the animal model. After 4 weeks of thioacetamide (TAA) administration mice showed distortion of liver architecture and regenerative nodules (Fig. 10). To assess the extent of liver fibrosis, formalin-fixed liver samples were embedded in paraffin and 5 µm sections were stained by Sirius Red for detection of fibrillar collagen. Quantitative analysis of Sirius red stained area was performed by computerized morphometric analysis. About 50 light microscope images (100X magnification) per specimen were captured and analyzed using the color threshold detection system developed in ImageJ software (NIH, USA). The results were expressed as percentage of positive area. Collagen deposition was quantified by morphometric analysis and densitometry.

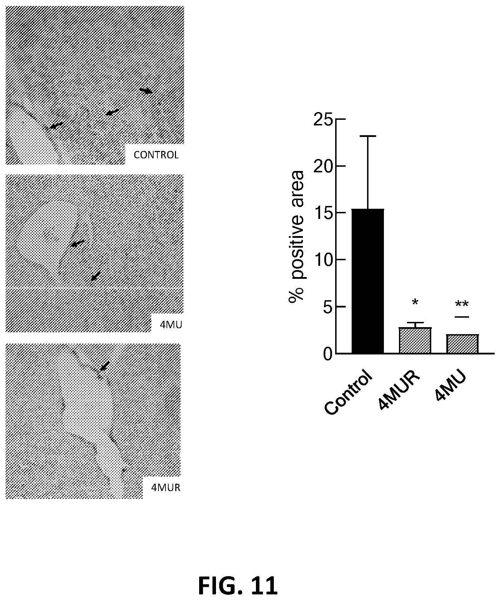

To further understand the 4MUR effect on HCC, HA expression was evaluated in liver tissue sections of mice from the different groups by staining with an HA-binding protein (HA-BP). Negative controls were stained with bHA-BP and pretreated with 100 U/ml Streptomyces hyaluronidase (# 385931, Calbiochem) at 37°C for 30 min. Peroxidase complex (Sigma) 1:10 in PBS was used for developing. The signal was detected by 0.1% diaminobenzidine (Sigma-Aldrich, Buenos Aires, Argentina), 4% glucose, 0.08% CINH₄, 5% nickel ammonium sulfate in 0.2 M AcNa and 0.05% H₂O₂. High HA expression was observed in liver sections from mice with both HCC and fibrosis. As a result of 4MUR therapy, mice showed a remarkable reduction in HA expression (Fig. 11).

### Example 6 - Toxicity study

In order to evaluate the toxicity of the compound of the invention, an *in vivo* assay with mice was carried out. Animals treated with 4MUR were kept healthy during all the experimental protocol.

Briefly, C3Hj mice untreated (n=4) or treated with 4MU (n=4) or 4MUR (n=5) were used to assess its toxicology profile. Mice weight was supervised for more than 20 days. At the end of the experiments, healthy mice or mice receiving 4MU/4MUR were sacrificed to collect liver, spleen, heart, lung, and kidney samples. Tissue sections from liver, spleen, heart, and lungs were paraffin-embedded and H&E staining procedure was performed using standard protocols.

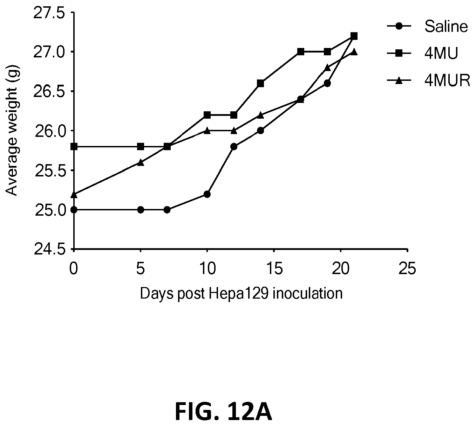

Animals' weight was recorded during the experimental assay. **Fig. 12A** shows the weight curve obtained. There were no significant changes noticed for animal body weight for both treatments.

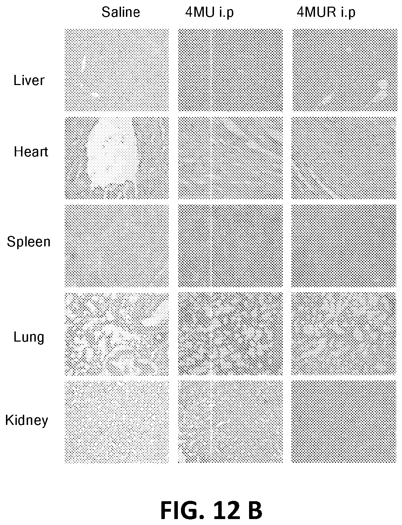

It could be seen that the treatment was well tolerated, i.e., without evident clinical toxicity signs according to the histology analysis with hematoxylin/eosin staining in hepatic tissue, kidney, lungs, spleen, and heart. As may be seen in **Fig. 12B** showing representative H&E stained tumor regions (magnification 20×) for treatments with either 4MU or 4MUR, there is no induced tissue damage. There are no necrotic zones observed, nor loss of tissue architecture in any organ.

Furthermore, 4MUR significantly reduced the amount of ascitic fluid when compared with untreated mice and 4MU (see Table 3 below).

**Table 3: Ascitic fluid accumulated in animals during the murine experimental assay**

| Mouse | Control (saline) | 4MU (20 mg/kg) | 4MUR (20mg(kg) |
|---|---|---|---|
| 1 | ++++ | +++ | - |
| 2 | +++ | ++ | - |
| 3 | +++ | ++++ | + |
| 4 | ++++ | ++ | - |
| 5 | ++++ | + | - |

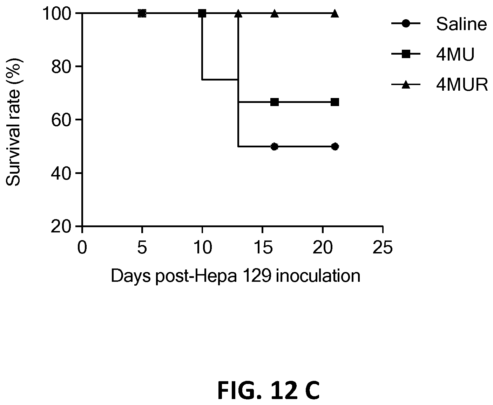

Finally, as shown in Fig. **12C****,** animal survival receiving 4MUR treatment (n=5) increased significantly as compared to mice receiving 4MU treatment or saline solution (control) (* p<0.05; Log-Rank test). It is important to notice that 4MUR treatment resulted in 100% survival, compared to 67% in 4MU and 50% in the control group.

## Claims

1. Pharmaceutical composition comprising rutinosyl-4-methylumbelliferone and pharmaceutically acceptable excipients, for use in the treatment of hepatocellular carcinoma (HCC).

## Patentansprüche

1. Pharmazeutische Zusammensetzung, die Rutinosyl-4-methylumbelliferon und pharmazeutisch verträgliche Hilfsstoffe umfasst, zur Verwendung bei der Behandlung von Leberzellkarzinomen (HCC).

## Revendications

1. Composition pharmaceutique comprenant du rutinosyl-4-methylumbelliférone et des excipients pharmaceutiquement acceptables, destinée à être utilisée dans le traitement du carcinome hépatocellulaire.
